(19) Europäisches Patentamt / European Patent Office / Office européen des brevets

(11) **EP 4 501 364 A1**

(12) **EUROPEAN PATENT APPLICATION**
published in accordance with Art. 153(4) EPC

(43) Date of publication:
**05.02.2025 Bulletin 2025/06**

(21) Application number: **23778991.2**

(22) Date of filing: **16.02.2023**

(51) International Patent Classification (IPC):
***A61K 50/00*** (2006.01)    ***C09K 3/00*** (2006.01)

(52) Cooperative Patent Classification (CPC):
**A61K 9/0009; C09K 3/00**

(86) International application number:
**PCT/JP2023/005602**

(87) International publication number:
**WO 2023/188961 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.03.2022  JP 2022052960**

(71) Applicant: **Sekisui Kasei Co., Ltd.**
**Kita-ku**
**Osaka-shi**
**Osaka 530-8565 (JP)**

(72) Inventor: **IIZUKA, Ryo**
**Osaka-shi, Osaka 530-8565 (JP)**

(74) Representative: **Müller-Boré & Partner**
**Patentanwälte PartG mbB**
**Friedenheimer Brücke 21**
**80639 München (DE)**

(54) **HYDROGEL**

(57)    An object of the present invention is to provide a hydrogel in which adhesion variation is suppressed while moderate adhesion to the skin is maintained. Provided are a hydrogel comprising a hydrogel and fibers dispersed in the hydrogel, the hydrogel containing no intermediate substrate, and the hydrogel having an adhesive force to a Bakelite plate of 1.0 to 10 N/20 mm and a coefficient of variation CV value of the adhesive force of less than 4.0, and a method for producing the same.

## Description

Technical Field

The present invention relates to a hydrogel.

Background Art

Hydrogels are suitably used as a surgical tape to be attached to a living body, a fixing tape for various medical instruments, a bioelectrode pad to be attached to a living body, an electrocardiogram electrode, a building material, an industrial adhesive tape for electronic materials, and the like. In these hydrogels, an intermediate substrate is often embedded for the purpose of reinforcement and/or improvement in shape retention at the time of cutting, and the like.
For example, PTL 1 discloses a gel sheet including an intermediate substrate embedded in a gel, in which the degree of waviness inside the gel sheet in the intermediate substrate is within a specific range.

Citation List

Patent Literature

PTL 1: JP 2020-97216 A

Summary of Invention

Technical Problem

However, a conventional gel sheet including an intermediate substrate may have a problem that adhesion to skin varies. If there is a variation in the adhesion, for example, when the gel sheet is used as an electrode gel pad, there is a disadvantage that the pad may be peeled off from the skin.
An object of the present invention is to provide a hydrogel in which adhesion variation is suppressed while moderate adhesion to the skin and processability/handling are maintained.

Solution to Problem

In order to solve the above problems, the inventors have extensively conducted studies, and resultantly found that when a fiber is contained instead of the intermediate substrate, adhesion variation can be suppressed while appropriate adhesion to the skin is maintained.
The present invention has been completed based on these findings, and includes inventions of the following broad aspects.

[Item 1]

A hydrogel comprising a hydrogel and fibers dispersed in the hydrogel,

the hydrogel comprising no intermediate substrate, and
the hydrogel having an adhesive force to a Bakelite plate of 1.0 to 10 N/20 mm and a coefficient of variation CV value of the adhesive force of less than 4.0.

[Item 2]

The hydrogel according to item 1, in which the fiber contains dietary fiber.

[Item 3]

The hydrogel according to item 1 or 2, in which the fiber contains dietary fiber and polyolefin fiber.

[Item 4]

The hydrogel according to item 2 or 3, in which a content of the dietary fiber is 0.20 to 2.0% by mass in terms of pure content.

[Item 5]

The hydrogel according to any one of items 2 to 4, in which the dietary fiber has an average fiber diameter of 10 to 500 nm.

[Item 6]

The hydrogel according to any one of items 2 to 5, in which the dietary fiber contains cellulose.

[Item 7]

The hydrogel according to item 3, in which a content of the polyolefin fiber is 0.20 to 2.0% by mass in terms of pure content.

[Item 8]

The hydrogel according to any one of items 3 to 7, in which the polyolefin fiber has an average fiber length of 0.50 to 2.0 mm.

[Item 9]

The hydrogel according to any one of items 1 to 8, which has an adhesive force to a Bakelite plate of 2.5 to 10 N/20 mm.

[Item 10]

The hydrogel according to any one of items 1 to 9, comprising a polymer matrix, water, and a humectant, and the polymer matrix being a copolymer of at least one monofunctional monomer selected from the group consisting of a (meth)acrylamide monomer and a (meth)acrylic acid ester, and a crosslinkable monomer.

[Item 11]

The hydrogel according to any one of items 1 to 10, which has a thickness of 0.20 to 2.0 mm.

[Item 12]

The hydrogel according to any one of items 1 to 11, which has a storage modulus of 3,000 to 9,000 Pa.

[Item 13]

A biological electrode hydrogel to be placed between an electrode comprising a conductive material and a skin surface, the biological electrode hydrogel comprising the hydrogel according to any one of items 1 to 12.

[Item 14]

A method for producing the hydrogel according to any one of items 1 to 12, comprising irradiating a polymerizable solution with ultraviolet light having a peak intensity of 50 to 150 mW/cm$^2$, the polymerizable solution comprising a monofunctional monomer, a crosslinkable monomer, a fiber, water, a polymerization initiator, and a humectant and having a crosslinkable monomer content of 0.010 to 0.050% by mass.

Advantageous Effects of Invention

According to the present invention, it is possible to provide a hydrogel in which adhesion variation is suppressed while moderate adhesion to the skin and processability/handling are maintained.

Description of Embodiments

As used herein, the singular forms (a, an, the, etc.) are intended to include singular and plural forms, unless the context indicates otherwise or clearly dictates otherwise.

In the present specification, "comprise" is a concept also including "consist essentially of" and "consist of".

In the numerical range described in stages in the present specification, the upper or lower limit of the numerical range at one stage can be optionally combined with the upper or lower limit of the numerical range at another stage. Also, in the numerical range described in the present specification, the upper or lower limit of the numerical range may be replaced with a value shown in Examples or a value that can be uniquely derived from Examples. Further, in the present specification, the numerical values connected by "to" mean the numerical range including the numerical values before and after "to" as the lower limit and the upper limit.

In the present specification, (meth)acryl means acryl or methacryl, and (meth)acrylate means acrylate or methacrylate.

Hydrogel

The hydrogel of the embodiment of the present invention is a hydrogel containing a hydrogel and fibers dispersed in the hydrogel. In addition, the hydrogel of the embodiment of the present invention contains no intermediate substrate, has an adhesive force to a Bakelite plate of 1.0 to 10 N/20 mm, and has a coefficient of variation CV value of the adhesive force of less than 4.0.

The hydrogel of the present invention is obtained by molding a hydrogel composition containing various components of the hydrogel and fibers into a sheet. As a component of the hydrogel composition for forming a hydrogel, a component used for a known hydrogel can be used. For example, the hydrogel composition contains a polymer matrix, water, and a humectant. Also, the hydrogel composition may further contain an electrolyte and one or more additives.

1. Polymer matrix

The polymer matrix is contained in an amount of preferably 10 to 60% by mass, more preferably 13 to 40% by mass, and still more preferably 15 to 35% by mass in 100% by mass of the hydrogel (hereinafter, "100% by mass of the hydrogel" may be paraphrased as 100% by mass of the hydrogel composition). When the content is 10% by mass or more, the strength of the hydrogel is increased, and the sheet shape is easily retained, which is preferable. Also, when the content is 60% by mass or less, it is preferable from the viewpoint of allowing movement of ions in the hydrogel.

The polymer matrix can be formed from a copolymer of a monofunctional monomer having one ethylenically unsaturated group, and a crosslinkable monomer.

1-1. Monofunctional monomer

The monofunctional monomer is not particularly limited as long as it has one ethylenically unsaturated group, but is preferably a water-soluble monomer. Examples of the water-soluble monomer include (meth)acrylamide monomers, (meth)acrylic acid esters, and the like.

Examples of the (meth)acrylamide monomer include N,N-dialkyl(meth)acrylamides such as (meth)acrylamide, N,N-dimethyl(meth)acrylamide, and N,N-diethyl(meth)acrylamide, N-alkyl(meth)acrylamides such as N-isopropyl(meth) acrylamide, N-methyl (meth) acrylamide, N-ethyl(meth)acrylamide, and N-propyl(meth)acrylamide; N-hydroxyalkyl(meth)acrylamides such as N-hydroxyethyl(meth)acrylamide and N-hydroxymethyl (meth)acrylamide; N-alkoxyalkyl(meth)acrylamides such as N-ethoxymethyl(meth)acrylamide, N-propoxymethyl(meth)acrylamide, N-butoxymethyl (meth)acrylamide, N-isobutoxymethyl(meth)acrylamide, N-pentoxymethyl(meth)acrylamide, N-hexyloxymethyl(meth) acrylamide, N-heptoxymethyl (meth)acrylamide, N-octoxymethyl(meth)acrylamide, N-ethoxyethyl (meth)acrylamide, N-propoxyethyl(meth)acrylamide, and N-butoxyethyl(meth)acrylamide; amino group-containing cationic acrylamide-based compounds such as dimethylaminopropyl(meth)acrylamide; sulfonic acid group-containing anionic monofunctional monomers such as 4-acryloylmorpholine and tert-butyl acrylamide sulfonic acid or salts thereof; derivatives thereof; and the like. Among them, at least one selected from the group consisting of (meth)acrylamide, N,N-dimethyl (meth) acrylamide, N,N-diethyl(meth)acrylamide, N-isopropyl (meth)acrylamide, N-methyl(meth)acrylamide, N-ethyl (meth) acrylamide, N-propyl(meth)acrylamide, N-hydroxyethyl (meth)acrylamide, N-hydroxymethyl(meth)acrylamide, dimethylaminopropyl(meth)acrylamide, 4-acryloylmorpholine, tert-butyl acrylamide sulfonic acid and salts thereof, and the like are preferably used, but not limited thereto.

Examples of the (meth)acrylic acid ester include (meth)acrylic acid alkyl esters in which the alkyl group has 1 to 18 carbon atoms, for example, (meth)acrylic acid alkyl esters, such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-hexyl (meth)acrylate, n-octyl (meth)acrylate, isooctyl (meth)acrylate, 2-ethylhexyl (meth)acrylate, n-nonyl (meth)acrylate, isononyl (meth)acrylate, n-pentyl (meth)acrylate, n-decyl (meth)acrylate, isodecyl (meth)acrylate, n-lauryl (meth)acrylate, tridecyl (meth)acrylate, and n-stearyl (meth)acrylate; alicyclic (meth)acrylic acid esters, such as cyclohexyl (meth) acrylate, isobornyl (meth)acrylate, and 1-adamantyl (meth)acrylate; alkoxy group-containing (meth)acrylic acid esters, such as 2-methoxyethyl (meth)acrylate, ethoxyethoxyethyl (meth)acrylate, and methoxypolyethyleneglycol (meth)acrylates such as methoxytriethyleneglycol (meth)acrylate; hydroxyalkyl (meth)acrylates (in which an aryl group may be bonded to the hydroxyalkyl group via an ether linkage), such as 2-hydroxyethyl (meth)acrylate, 2-hydroxypropyl (meth) acrylate, 2-hydroxy-3-phenoxypropyl (meth)acrylate, and 2-hydroxybutyl (meth)acrylate; glycerol mono(meth)acrylate; polyalkylene glycol mono(meth)acrylates, such as polyethylene glycol mono(meth)acrylate and a polyethylene glycol-polypropylene glycol copolymer; (meth)acrylic acid esters having an aromatic ring, such as benzyl (meth)acrylate; (meth) acrylic acid esters having a heterocyclic ring, such as tetrahydrofurfuryl (meth)acrylate; and the like. These monofunctional monomers may be used singly or in a combination of two or more.

As the monofunctional monomer, in addition to the (meth)acrylamide monomer and the (meth)acrylic acid ester, optionally, a vinylamide monofunctional monomer such as (meth)acrylic acid or a salt thereof, vinylpyrrolidone, vinylacetamide, or vinylformamide; a nonionic monofunctional monomer such as allyl alcohol, a styrene-based monomer, or the like can be used. These monofunctional monomers may be used singly or in a combination of two or more.

The content of the structural unit derived from the monofunctional monomer in the hydrogel is not particularly limited, but is preferably 8.5 to 60% by mass, more preferably 10 to 40% by mass, and still more preferably 15 to 35% by mass. If the content of the structural unit derived from the monofunctional monomer is within the above range, it is preferable from the viewpoint of the shape retention and flexibility of the hydrogel. If the content is 8.5% by mass or more, the hydrogel has sufficient shape retention, and there is little possibility that the hydrogel is too soft or easily torn. In addition, if the content is 60% by mass or less, the flexibility of the hydrogel is hardly impaired, and it is preferable from the viewpoint of allowing the movement of ions in the hydrogel.

1-2. Crosslinkable monomer

As the crosslinkable monomer, it is preferable to use a monomer having two or more double bonds having polymerizability in the molecule. Examples of the crosslinkable monomer include polyfunctional (meth)acrylamides or (meth)acrylates, such as methylenebis(meth)acrylamide, ethylenebis(meth)acrylamide, (poly)ethylene glycol di(meth) acrylate, (poly)propylene glycol di(meth)acrylate, glycerol di(meth)acrylate, and glycerol tri(meth)acrylate; tetraallyloxyethane; diallyl ammonium chloride; and the like, and these can be used alone or in a combination of two or more. As the crosslinkable monomer having two or more double bonds having polymerizability in the molecule, a polyglycerol derivative that is a polyfunctional compound having two or more (meth)acryloyl groups or vinyl groups and having a molecular weight of 400 or more, described in JP 2803886 B1, can also be used.

The content of the structural unit derived from the crosslinkable monomer in the hydrogel is preferably within the range of 0.010 to 1.5% by mass, more preferably 0.010 to 0.30% by mass, and still more preferably 0.010 to 0.050% by mass. If the content of the structural unit derived from the crosslinkable monomer is within the above range, it is preferable from the viewpoint of the shape, adhesive force, handleability, and flexibility of the hydrogel. If the content is 0.010% by mass or more, a concern about deterioration of shape stability due to a low crosslinking density hardly occurs, and the cohesive force and the holding force of the hydrogel itself are not reduced, so that a hydrogel having an appropriate adhesive force can be obtained. In addition, a concern that the handleability of the hydrogel is deteriorated, for example, a part of the gel material remaining on the adherend at the time of peeling, also hardly occurs. When the content is 1.5% by mass or less, a hydrogel having an appropriate adhesive force is obtained, and the flexibility of the hydrogel is hardly impaired.

2. Water

The content of water in the hydrogel is not particularly limited, but is preferably 10 to 60% by mass, more preferably 10 to 45% by mass, and still more preferably 15 to 30% by mass. If the content of water is 10% by mass or more, the water content with respect to the equilibrium water content of the hydrogel is not too low, and deterioration derived from hygroscopicity (for example, swelling or the like) of the hydrogel hardly occurs. In addition, if the content of water is 60% by mass or less, the water content with respect to the equilibrium water content of the hydrogel is not too large, and deterioration derived from drying (for example, contraction or the like) of the hydrogel hardly occurs.

3. Humectant

The humectant is not particularly limited, and examples thereof include diols, such as ethylene glycol, triethylene glycol, 1,6-hexanediol, 1,9-nonanediol, propylene glycol, and butanediol; tri- or higher polyhydric alcohols, such as glycerol, pentaerythritol, and sorbitol; polyhydric alcohol condensates, such as polyethylene glycol, polypropylene glycol, and polyglycerol; modified polyhydric alcohols, such as polyoxyethylene glycerol; polyoxyethylene alkyl ethers, such as polyoxyethylene lauryl ether, polyoxyethylene stearyl ether, polyoxyethylene isostearyl ether, and polyoxyethylene methyl glucoside, polyoxyalkylene alkyl ethers, such as polyoxypropylene alkyl ethers, such as polyoxypropylene lauryl ether, polyoxypropylene stearyl ether, polyoxypropylene isostearyl ether, and polyoxypropylene methyl glucoside; and the like.

Among the humectants, it is preferable to use a polyhydric alcohol that is liquid in a use temperature range of the hydrogel (for example, around 20°C when used indoors), and specifically, ethylene glycol, triethylene glycol, propylene glycol, polypropylene glycol, polyethylene glycol, polyglycerol, glycerol, and the like are suitable.

The content of the humectant in the hydrogel is not particularly limited, but is preferably 20 to 70% by mass, and more preferably 25 to 65% by mass. If the content of the humectant is 20% by mass or more, the hydrogel to be obtained has a moisturizing power, and evaporation of moisture is also suppressed, so that temporal stability of the hydrogel is improved, which is preferable. In addition, if the content of the humectant is 70% by mass or less, the humectant does not bleed out from the hydrogel surface, and a decrease in adhesive force due to the bleed out is suppressed, which is preferable.

### 4. Electrolyte

The hydrogel can optionally contain an electrolyte, which can impart conductivity to the hydrogel.

In the case of imparting conductivity to the hydrogel, the content of the electrolyte in the hydrogel is preferably 0.050 to 10% by mass, and more preferably 0.10 to 6.0% by mass. If the content of the electrolyte is 0.05% by mass or more, impedance of the hydrogel decreases, and conductivity is good, which is preferable. In addition, the impedance decreases as the content of the electrolyte increases, but if the content of the electrolyte is too large, the impedance no longer decreases, which is wasteful in terms of cost. Therefore, the content of the electrolyte is preferably 10% by mass or less from the viewpoint of balance between conductivity and cost.

The electrolyte is not particularly limited, and examples thereof include alkali metal halides such as sodium halides (for example, sodium chloride), lithium halides, and potassium halides; alkaline earth halides such as magnesium halides and calcium halides; other metal halides; and the like. In addition, as the electrolyte, hypochlorites, chlorites, chlorates, perchlorates, hydrochlorides, sulfates, carbonates, nitrates, and phosphates of various metals are also suitably used. Moreover, as the electrolyte, inorganic salts, such as ammonium salts and complex salts; salts of monovalent organic carboxylic acids, such as acetic acid, benzoic acid, and lactic acid; salts of polyvalent organic carboxylic acids, such as tartaric acid; monovalent, divalent, or higher valent salts of polyvalent carboxylic acids, such as phthalic acid, succinic acid, adipic acid, and citric acid; metal salts of organic acids, such as sulfonic acids and amino acids; organic ammonium salts; and the like are also preferable.

Further, a base such as sodium hydroxide may be appropriately added to the hydrogel for the purpose of adjusting the pH.

### 5. Additive

Furthermore, the hydrogel may optionally contain other additives. Examples of the other additives include rust inhibitors, antifungal agents, antioxidants, antifoaming agents, stabilizers, surfactants, colorants, essential oils, and the like.

### 6. Fiber

The fiber contained in the hydrogel is not particularly limited as long as it can be dispersed in the hydrogel, and may be a hydrophilic fiber or a hydrophobic fiber, but a hydrophilic fiber is preferable. It can also be said that the fibers are dispersed in the hydrogel composition or the hydrogel. Also, the fibers are preferably uniformly dispersed in the hydrogel composition or the hydrogel.

Examples of the hydrophilic fiber include dietary fibers such as cellulose; artificial cellulose fibers such as rayon and acetate; hydrophilized polyolefin fibers, synthetic fibers such as polyamide and polyester, and the like. These fibers may be used alone or in combination of two or more, but it is preferable to use a combination of two or more. In addition, it is preferable to contain at least dietary fibers as fibers, and it is more preferable to contain dietary fibers and polyolefin fibers.

The content of the fiber is preferably 0.50 to 2.5% by mass in total in terms of pure content, and is preferably 0.60 to 2.0% by mass. If the content of the fiber is 0.50% by mass or more, the mechanical strength and hardness of the hydrogel are good, which is preferable. If the content of the fiber is 2.5% by mass or less, the hydrogel is not too hard, and the flexibility is good, which is preferable.

6-1. Dietary fiber

The dietary fiber is not particularly limited as long as it can be added to the hydrogel and used. Examples of the dietary fiber include cellulose, polydextrose, sodium alginate, inulin, carrageenan, hemicellulose, lignin, chitin, chitosan, pectin, and the like. Also, examples of commercially available products include EXILVA (manufactured by Borregaard), Herbacel AQ Plus (registered trademark), Fiberlon (registered trademark), Healthy Gum (registered trademark) (as described above, manufactured by Sumitomo Pharma Food & Chemical Co., Ltd.), and the like. In particular, cellulose is preferred.

The cellulose is not particularly limited as long as it can be added to the hydrogel and used. Examples of the cellulose include those derived from plant raw materials such as wood fibers (pulp), those in which some of hydroxyl groups are oxidized, and those chemically modified by etherification such as acylation and carboxymethylation, esterification, and other reactions, and the like. The cellulose is obtained, for example, by appropriately subjecting a raw material to a chemical treatment such as oxidation or etherification, and then pulverizing the raw material by a mechanical method or the like into a fibrous form.

The cellulose may contain one or more of lignocellulose, cellulose nanofiber (CNF), cellulose nanocrystal (CNC), and microfibrillated cellulose (NFC). Lignocellulose is composed of cellulose, hemicellulose, and lignin. Cellulose nanofibers, cellulose nanocrystals, and the like are obtained by defibrating cellulose fibers contained in wood pulp or the like to a nano-size level. Microfibrillated cellulose contains cellulose microfibrils (single cellulose nanofibers) which are basic skeletal substances of plants.

The dietary fiber preferably has an average fiber diameter of 10 to 500 nm, and more preferably 20 to 300 nm. If the dietary fiber has an average fiber diameter of 10 nm or more, the mechanical strength of the hydrogel is good, which is preferable. In addition, if the dietary fiber has an average fiber diameter of 500 nm or less, the interaction between the dietary fiber and the polymer matrix is good and the mechanical strength is good, which is preferable. The average fiber diameter of the dietary fibers can be determined by measuring fiber diameters at 10 or more arbitrary points by morphological observation with a scanning electron microscope and calculating the number average thereof.

The average fiber length of the dietary fibers is not particularly limited, but is preferably 1.0 to 50 $\mu$m, and more preferably 5.0 to 30 $\mu$m. If the dietary fiber has an average fiber length of 1.0 $\mu$m or more, the mechanical strength of the hydrogel is improved and handleability is good, which is preferable. Also, if the dietary fiber has an average fiber length of 50 $\mu$m or less, dispersibility of the dietary fiber in the hydrogel is good, which is preferable. The average fiber length of the dietary fibers can be determined by measuring fiber lengths at 10 or more arbitrary points by morphological observation with a scanning electron microscope and calculating the number average thereof.

The content of the dietary fiber is preferably 0.20 to 2.0% by mass and more preferably 0.50 to 1.5% by mass in terms of pure content. If the content of the dietary fiber is 0.20% by mass or more, the mechanical strength and hardness of the hydrogel are good, which is preferable. If the content of the dietary fiber is 2.0% by mass or less, the hydrogel is not too hard, has moderate adhesion, and has good flexibility, which is preferable.

6-2. Polyolefin fiber

Examples of the polyolefin fiber include polyethylene fibers, polypropylene fibers, and the like. The polyolefin fiber is preferably hydrophilized from the viewpoint of miscibility with the hydrogel, dispersion stability, or miscibility and dispersion stability in the hydrogel. The hydrophilization treatment is not particularly limited, but the hydrophilization treatment is performed by application of a hydrophilization treatment agent such as a surfactant, surface modification by corona surface treatment or plasma discharge treatment, or the like. As the hydrophilized polyolefin fiber, a commercially available product can be used, and examples thereof include SWP series (manufactured by Mitsui Chemicals, Inc.) and the like.

The polyolefin fiber has an average fiber diameter of preferably 1.0 to 60 $\mu$m, and more preferably 5.0 to 30 $\mu$m. If the polyolefin fiber has an average fiber diameter of 1.0 $\mu$m or more, the polyolefin fiber has rigidity and the mechanical strength of the hydrogel is good, which is preferable. In addition, if the polyolefin fiber has an average fiber diameter of 60 $\mu$m or less, the interaction between the polyolefin fiber and the polymer matrix is good, and the mechanical strength is good, which is preferable. The average fiber diameter of the polyolefin fibers can be determined by measuring fiber diameters at 10 or more arbitrary points by morphological observation with a scanning electron microscope and calculating the number average thereof.

The polyolefin fiber has an average fiber length of preferably 0.50 to 2.0 mm, and more preferably 0.90 to 1.5 mm. If the polyolefin fiber has an average fiber length of 0.50 mm or more, the mechanical strength of the hydrogel is improved, and handling is good, which is preferable. If the polyolefin fiber is 2.0 mm or less, dispersibility of the polyolefin fiber in the hydrogel is good, which is preferable. The average fiber length of the polyolefin fibers can be determined by measuring the fiber lengths of a plurality of (for example, 10 or more) fibers with an optical microscope and calculating the number average thereof.

The content of the polyolefin fiber is preferably 0.20 to 2.0% by mass and more preferably 0.50 to 1.5% by mass in terms of pure content. If the content of the polyolefin fiber is 0.20% by mass or more, the mechanical strength and hardness of the hydrogel are good, which is preferable. If the content of the polyolefin fiber is 2.0% by mass or less, the hydrogel is not too hard, and the flexibility is good, which is preferable.

Method for producing hydrogel

The hydrogel of the present invention can be produced by a method including the following Step 1 and Step 2.
(Step 1) Preparing a polymerization solution containing a monofunctional monomer, a crosslinkable monomer, a fiber, water, a polymerization initiator, and a humectant.
(Step 2) Polymerizing the polymerization solution.

Step 1

It is preparing a polymerization solution containing a monofunctional monomer, a crosslinkable monomer, a fiber, water, a polymerization initiator, and a humectant. For the preparation of a polymerization solution, the above-mentioned materials can be used. A polymerization solution is prepared by uniformly dispersing these materials.
As the polymerization initiator, both a thermal polymerization initiator and a photopolymerization initiator can be used, but it is preferable to use a photopolymerization initiator having small change in components before and after polymerization.
Examples of the photopolymerization initiator include 2-hydroxy-2-methyl-1-phenyl-propan-1-one (product name: Omnirad 1173, manufactured by IGM Resins B.V.), 1-hydroxy-cyclohexyl-phenyl-ketone (product name: Omnirad 184, manufactured by IGM Resins B.V.), 1-[4-(2-hydroxyethoxy)-phenyl]-2-hydroxy-2-methyl-propan-1-one (product name: Omnirad 2959, manufactured by IGM Resins B.V.), 2-methyl-1-[(methylthio)phenyl]-2-morpholinopropan-1-one (product name: Omnirad 907, manufactured by IGM Resins B.V.), 2-benzyl-2-dimethylamino-1-(4-morpholinophenyl)-butan-1-one (product name: Omnirad 369, manufactured by IGM Resins B.V.), and the like. The polymerization initiators may be used singly or in a combination of two or more.
The amount of the polymerization initiator used is preferably 0.010 to 3.0% by mass, and more preferably 0.050 to 1.0% by mass, based on 100% by mass of the total of all monomers (monofunctional monomer and crosslinkable monomer). If the content is 0.010% by mass or more, no unpolymerized monomer remains in the hydrogel, and if the content is 3.0% by mass or less, there is no concern about odor and/or discoloration (yellowing) due to the residue of the polymerization initiator after the polymerization reaction, which is preferable.
Preferred mixing ratio of each component in the polymerization solution is as follows.

- Monofunctional monomer: 10 to 60% by mass, more preferably 15 to 35% by mass.
- Crosslinkable monomer: 0.010 to 1.5% by mass, more preferably 0.010 to 0.050% by mass.
- Fiber: 0.50 to 2.5% by mass, more preferably 0.60 to 2.0% by mass in terms of pure content.
- Water: 10 to 60% by mass, more preferably 15 to 30% by mass.
- Humectant: 20 to 70% by mass, more preferably 25 to 65% by mass.
- Polymerization initiator: 0.010 to 3.0% by mass, more preferably 0.050 to 1.0% by mass.
- Electrolyte: 0.050 to 10% by mass, more preferably 0.1 to 6.0% by mass.
- Other components: 0.50 to 2.0% by mass.

In the above, the fiber preferably contains a dietary fiber. The mixing ratio of the dietary fiber is preferably 0.20 to 2.0% by mass and more preferably 0.50 to 1.5% by mass in terms of pure content. Also, the fiber preferably contains a polyolefin fiber. The mixing ratio of the polyolefin fiber is preferably 0.20 to 2.0% by mass and more preferably 0.50 to 1.5% by mass in terms of pure content.
The polymerization solution is prepared by sufficiently stirring until the components are uniformly dispersed. The temperature during stirring is preferably 20 to 40°C.
The polymerization solution is optionally formed into a sheet after preparation and before polymerization in Step 2. Examples of forming of the polymerization solution into a sheet include (a) a method of injecting the polymerization solution into a mold, (b) a method of pouring the polymerization solution between protective films and retaining the obtained product at a constant thickness, (c) a method of coating the polymerization solution on a protective film, and the like. The method (a) has an advantage that a hydrogel having an arbitrary shape can be obtained. The methods (b) and (c) have an advantage that a relatively thin hydrogel can be obtained.
As the protective film, for example, a resin film made of a resin such as polyester, polyolefin, polystyrene, or polyurethane, paper, paper obtained by laminating the resin film, or the like can be used.
The side of the protective film in contact with the polymerization solution or the hydrogel has been preferably release-treated. Examples of the method for the release treatment include silicone coating, and the like, and in particular, baking-type silicone coating in which crosslinking and curing reactions are performed by heat or ultraviolet light is preferable. The film subjected to the release treatment is particularly preferably a biaxially stretched PET (polyethylene terephthalate) film, an OPP (stretched polypropylene) film, or the like.

Further, in a case where polymerization is performed in a state in which the protective film is provided, by, for example, irradiating ultraviolet light from above the protective film in the following Step 2, it is preferable to select a film of a material that does not block light in order not to hinder photopolymerization.

Step 2

A hydrogel can be obtained by polymerizing the polymerization solution by heat application or light irradiation. The conditions for the heat application and the light irradiation are not particularly limited as long as a hydrogel can be obtained, and general conditions can be adopted.

When polymerization is performed by ultraviolet irradiation, the integrated amount of ultraviolet irradiation varies also depending on, for example, the content of the polymerization initiator. For example, the integrated amount of ultraviolet irradiation is preferably within the range of 1,000 mJ/cm$^2$ to 10,000 mJ/cm$^2$, and more preferably within the range of 2,000 mJ/cm$^2$ to 10,000 mJ/cm$^2$. Also, the peak intensity of the ultraviolet light is preferably within the range of 30 mW/cm$^2$ to 300 mW/cm$^2$, more preferably within the range of 50 mW/cm$^2$ to 150 mW/cm$^2$, and particularly preferably within the range of 70 mW/cm$^2$ to 130 mW/cm$^2$. By setting the amount of the polymerization initiator and the amount of ultraviolet irradiation appropriately, the reaction rate of the monofunctional monomer and the crosslinkable monomer can be suitably adjusted.

Physical properties and the like of hydrogel

The thickness of the hydrogel of the present invention is appropriately selected according to the application, and is, for example, within the range of 0.20 to 2.0 mm. In a preferred embodiment, the thickness of the hydrogel of the present invention is 0.30 to 1.2 mm.

The adhesive force of the hydrogel of the present invention to a Bakelite plate is 1.0 to 10 N/20 mm. It is preferably 2.5 to 10 N/20 mm, and more preferably 3.0 to 10 N/20 mm. If the adhesive force is less than 1.0 N/20 mm, the hydrogel may fall off from the skin. When the adhesive force is more than 10 N/20 mm, the adhesive force to the skin is too strong, and pain and/or redness may be accompanied at the time of peeling.

In addition, the coefficient of variation CV value of the adhesive force of the hydrogel of the present invention to a Bakelite plate is less than 4.0. It is preferably less than 3.8, and more preferably less than 3.5. The coefficient of variation CV value can be used as an indicator of variation in adhesive force. If the coefficient of variation is 4.0 or more, variations in adhesive force are large, and partial floating of the hydrogel during use and/or falling of the hydrogel from the skin easily occur.

In the present invention, the adhesive force to a Bakelite plate and the coefficient of variation of the adhesive force are measured by the following method.

The hydrogel is cut to a size of 120 mm × 20 mm and attached to a Bakelite plate, followed by pressing by reciprocating a 2-kg press roller once, to obtain a test piece. A rheometer (CR-500DX manufactured by Sun Scientific Co., Ltd.) is used as a measuring instrument, and measurement is performed at an angle of 90 degrees and a speed of 300 mm/min. Stress values (N/20 mm) at predetermined peeling-off points (30, 40, 50, 60, 70 mm) from the measurement starting point in the test piece are used as measured values, and the adhesive force can be calculated from the number average of the measured values. In addition, the coefficient of variation CV value can be calculated from the measured values by the following formula.

$$\text{CV Value (\%)} = (\text{Standard deviation} \times 100)/\text{Average value}$$

The hydrogel of the present invention has a storage modulus of preferably 3,000 Pa to 9,000 Pa, and more preferably 4,000 Pa to 8,000 Pa. The higher the storage modulus is, the more elastic it is, and the storage modulus serves as an indicator of hardness. If the storage modulus is 3,000 Pa or more, the hydrogel is not too soft, and the mechanical strength and handling of the hydrogel are good. If the storage modulus is 9,000 Pa or less, the hydrogel is not too hard, and the handling of the hydrogel is good. In the present invention, the storage modulus is measured by the following method.

Using a viscoelasticity measuring device (MR-102 manufactured by Anton Paar GmbH) as a measuring instrument, viscoelasticity is measured at 1% strain, 23°C, and a frequency of 0.1 Hz. A 25φ gel piece is attached to a 25φ SUS parallel plate, a jig is pressed against the gel piece to a load point of 1 N, and the storage modulus at 0.1 Hz is then calculated.

In some embodiments, the hydrogel is excellent in processability. When the content of the fiber is 0.50 to 2.5% by mass in terms of pure content, a hydrogel excellent in processability is obtained. Also, when the content of the dietary fiber is 0.20 to 2.5% by mass in terms of pure content, a hydrogel excellent in processability is obtained. Further, when the content of the polyolefin fiber is 0.20 to 2.0% by mass in terms of pure content, a hydrogel excellent in processability is obtained. Furthermore, when the content of the dietary fiber is 0.20 to 2.0% by mass in terms of pure content and the content of the polyolefin fiber is 0.20 to 2.0% by mass in terms of pure content, a hydrogel excellent in processability is obtained.

Application of hydrogel

Since the hydrogel of the present invention has no variation in adhesive force, it is suitably used for applications in which uniform adhesion is required. For example, it can be used as a wound covering material, a biological adhesive material, or a biological electrode hydrogel. Preferably, the hydrogel of the above embodiment of the present invention can be used as a biological electrode hydrogel to be placed between an electrode made of a conductive material and a skin surface.

In addition, the hydrogel of the present invention can be suitably used as a substrate of a transdermal absorbent by being impregnated with a chemical agent, as an electrode material for industrial measurement, an industrial adhesive material, or the like in industrial applications, as a non-polarized electrode for performing electrical geological survey, for example, by being installed on a surface of ground or rock in building material applications, as a conductive material for detecting breakage of a water shielding sheet used in a waste disposal site, or the like, as a conductive adhesive material installed between a concrete structure and a metal as an anode in an electrolytic corrosion preventing method for a concrete structure, or the like.

Examples

The present invention will be described in more detail using production examples and examples, but the present invention is not limited to these examples.

Example 1

1. Preparation of polymerization solution

As shown in Table 1, 20% by mass of acrylamide as a monofunctional monomer, 0.018% by mass of methylenebisacrylamide as a crosslinkable monomer, 0.50% by mass in terms of pure content of microfibrillated cellulose (Exilva, P-01V, manufactured by Borregaard) as a dietary fiber, 0.50% by mass in terms of pure content of polyethylene multibranched fiber (SWP, E790, manufactured by Mitsui Chemicals, Inc.) as a polyolefin fiber, 18% by mass of ion exchange water, and 58% by mass of glycerol as a humectant were added to a stirring and mixing container, and uniformly dispersed using a homomixer. Next, 2.0% by mass of sodium chloride as an electrolyte and 0.982% by mass of citric acid, Na benzoate, a photopolymerization initiator (Omnirad 2959, manufactured by IGM Resins B.V., 0.13% by mass), and a surfactant in total as other additives were added, and the mixture was stirred until completely dissolved to obtain a polymerization solution.

2. Production of hydrogel

The obtained polymerization solution was dropped on a PET film coated with silicone and allowed to pass through a certain clearance to spread the liquid uniformly, and a PET film coated with silicone was placed thereon to spread the liquid uniformly, followed by fixing so that the thickness was 0.75 mm. Ultraviolet irradiation was performed at a peak illuminance of 130 mW/cm$^2$ in an energy amount of 3,000 mJ/cm$^2$ using a metal halide lamp, thereby obtaining a hydrogel with a thickness of 0.75 mm.

Examples 2 to 8 and 10

Polymerization solutions were prepared in the same manner as in Example 1 except that the % by mass of each component and the thickness of the gel were changed as shown in Table 1. Hydrogels were produced using these mixture liquids in the same manner as in Example 1.

Example 9

As shown in Table 1, 14.4% by mass of acrylic acid as a monofunctional monomer and 9.6% by mass of tert-butyl acrylamide sulfonic acid (TBAS) as monofunctional monomers, 0.018% by mass of methylenebisacrylamide as a crosslinkable monomer, and 8.0% by mass of a 50% by mass NaOH solution were added to a stirring and mixing container to adjust the pH to 4 to 5. Thereafter, 0.50% by mass in terms of pure content of microfibrillated cellulose (Exilva, P-01V, manufactured by Borregaard) as a dietary fiber, 0.50% by mass in terms of pure content of polyethylene multibranched fiber (SWP, E790, manufactured by Mitsui Chemicals, Inc.) as a polyolefin fiber, 17.3% by mass of ion exchange water, and 46.7% by mass of glycerol as a humectant were added to a stirring and mixing container, and uniformly dispersed using a homomixer. Next, 2.0% by mass of sodium chloride as an electrolyte and 0.982% by mass of citric acid, Na benzoate, a photopolymerization initiator, and a surfactant in total as other additives were added, and the mixture was stirred until completely dissolved to obtain a polymerization solution. A hydrogel was produced using the polymerization solution in the same manner as in Example 1.

Example 11

As shown in Table 1, 20% by mass of acrylamide as a monofunctional monomer, 0.018% by mass of methylenebisacrylamide as a crosslinkable monomer, 0.50% by mass of a dietary fiber derived from citrus (Herbacel AQ Plus, manufactured by Sumitomo Pharma Food & Chemical Co., Ltd.) as a dietary fiber, 0.50% by mass in terms of pure content of polyethylene multibranched fiber (SWP, E790, manufactured by Mitsui Chemicals, Inc.) as a polyolefin fiber, 18% by mass of ion exchange water, and 58% by mass of glycerol as a humectant were added to a stirring and mixing container, and uniformly dispersed using a homomixer. Next, 2.0% by mass of sodium chloride as an electrolyte and 0.982% by mass of citric acid, Na benzoate, a photopolymerization initiator (Omnirad 2959, manufactured by IGM Resins B.V., 0.13% by mass), and a surfactant in total as other additives were added, and the mixture was stirred until completely dissolved to obtain a polymerization solution. A hydrogel was produced using the polymerization solution in the same manner as in Example 1.

Examples 12 and 13

Polymerization solutions were prepared in the same manner as in Example 11 except that the % by mass of each component and the thickness of the gel were changed as shown in Table 1. Hydrogels were produced using these mixture liquids in the same manner as in Example 1.

Comparative Examples 1 to 4

Polymerization solutions were prepared in the same manner as in Example 1 except that the % by mass of each component and the thickness of the gel were changed as shown in Table 2. Hydrogels were produced using these mixture liquids in the same manner as in Example 1.

However, since Comparative Example 1 had poor dispersibility and was phase-separated, no hydrogel was produced, and Comparative Example 1 was excluded from the evaluation target.

Comparative Example 5

1. Preparation of polymerization solution

As shown in the table, 20% by mass of acrylamide as a monofunctional monomer, 0.018% by mass of methylenebisacrylamide as a crosslinkable monomer, and 18% by mass of ion exchange water were mixed in a stirring and mixing container, and the mixture was stirred to be uniformly dissolved, then 59.0% by mass of glycerol was added as a humectant, and the mixture was uniformly dispersed using a homomixer. Next, 2.0% by mass of sodium chloride as an electrolyte and 0.982% by mass of citric acid, Na benzoate, a photopolymerization initiator, and a surfactant in total as other additives were added, and the mixture was stirred until completely dissolved. The mixture was stirred until it became uniform to obtain a polymerization solution.

2. Production of hydrogel

The obtained polymerization solution was dropped on a PET film coated with silicone and allowed to pass through a certain clearance to spread the liquid uniformly, and an intermediate substrate (nonwoven fabric) was placed thereon. The mixture liquid was further dropped thereon, and a PET film coated with silicone was placed thereon to spread the liquid uniformly, followed by fixing so that the thickness was 0.75 mm. Ultraviolet irradiation was performed at a peak illuminance of 130 mW/cm$^2$ in an energy amount of 3,000 mJ/cm$^2$ using a metal halide lamp, thereby obtaining a hydrogel with a thickness of 0.75 mm.

Comparative Example 6

A polymerization solution was prepared in the same manner as in Comparative Example 5. A hydrogel was produced in the same manner as in Comparative Example 5 except that the intermediate substrate was not used using the polymerization solution.

Comparative Example 7

As shown in Table 2, 14.4% by mass of acrylic acid as a monofunctional monomer and 9.6% by mass of tert-butyl acrylamide sulfonic acid (TBAS) as monofunctional monomers, 0.018% by mass of methylenebisacrylamide as a crosslinkable monomer, and 8.0% by mass of a 50% by mass NaOH solution were added to a stirring and mixing container to adjust the pH to 4 to 5. Thereafter, 17.3% by mass of ion exchange water and 47.7% by mass of glycerol as a humectant were added, and the mixture was stirred until complete dissolution. Next, 2.0% by mass of sodium chloride as an electrolyte and 0.982% by mass of citric acid, Na benzoate, a photopolymerization initiator, and a surfactant in total as other additives were added, and the mixture was stirred until completely dissolved to obtain a polymerization solution. A hydrogel was produced using the polymerization solution in the same manner as in Example 1.

Comparative Examples 8 to 10

A polymerization solution was prepared in the same manner as in Example 11 except that the % by mass of each component was changed as shown in Table 2. Hydrogels were produced using these mixture liquids in the same manner as in Example 11.
However, since Comparative Example 8 had poor dispersibility and was phase-separated, no hydrogel was produced, and Comparative Example 8 was excluded from the evaluation target.

Evaluation of adhesive force to Bakelite plate

The hydrogel was cut to a size of 120 mm × 20 mm, and the PET film was peeled off. A Bakelite plate was attached to the exposed gel surface, followed by pressing by reciprocating a 2-kg press roller once, to obtain a test piece. A rheometer (CR-500DX manufactured by Sun Scientific Co., Ltd.) was used for measurement, and the measurement was performed under the measurement conditions of an angle of 90 degrees and a speed of 300 mm/min. Stress values (N/20 mm) at predetermined peeling-off points (30, 40, 50, 60, 70 mm) from the measurement starting point were used as measured values. The adhesive force was calculated from the average value of the values of three tests (15 points in total). In addition, the coefficient of variation CV value was calculated from the values of three tests (15 points in total) by the following formula, and this value was used as an indicator of variation in the adhesive force of the hydrogel.

$$CV \text{ Value (\%)} = (\text{Standard deviation} \times 100)/\text{Average value}$$

The measurement was performed under an environment of a temperature of 23 ± 5°C and a humidity of 55% ± 10%. The results are shown in Tables 1 and 2.

Evaluation of Handling

The hydrogel was cut to a size of 120 mm × 20 mm, and the PET film was peeled off. An SUS plate (SUS304 mirror finished) was attached to the exposed gel surface, followed by pressing by reciprocating a 2-kg press roller once, to obtain a test piece. When 15 trained panels peeled off the hydrogel, the case where the gel did not stretch and was cleanly peeled off was rated as "A", the case where the gel was partially deformed but was cleanly peeled off was rated as "B", and the case where the gel stretched and was not cleanly peeled off was rated as "C". The results are shown in Tables 1 and 2.

Evaluation of processability

In order to prepare 16 test pieces of 40 mm × 40 mm, a Thomson blade was inserted up to the interface between the PET film and the hydrogel to cut only the hydrogel without cutting the PET film, thereby punching the test pieces. The gap between the test pieces was set to 8 mm. Then, the unnecessary hydrogel of the frame portion (margin portion) surrounding the punched test piece generated by the punching was peeled off. One in which even one test piece out of the 16 test pieces that was peeled off when the margin portion was peeled off, or one in which the cut surface of the test piece was rough at the time of cutting with a Thomson blade was determined to have a processability of "B" (poor), and one in which all of the 16 test pieces remained in a clean state was determined to have a processability of "A" (good). The results are shown in Tables 1 and 2.

Evaluation of storage modulus

Using a viscoelasticity measuring device (MR-102 manufactured by Anton Paar GmbH), viscoelasticity measurement was performed at 1% strain, 23°C, and a frequency of 0.1 Hz. A 25$\varphi$ gel piece was attached to a 25$\varphi$ SUS parallel plate, a jig was pressed against the gel piece to a load point of 1 N, and the storage modulus at 0.1 Hz was then calculated. The storage modulus serves as an indicator of hardness showing elasticity, as the storage modulus is higher. The results are shown in Tables 1 and 2.

Liquid stability

The polymerization solution was left standing for one day after preparation, and liquid stability was visually confirmed. A uniformly dispersed polymerization solution was rated as "A", a partially insufficiently dispersed polymerization solution was rated as "B", and a completely phase-separated polymerization solution was rated as "C". The results are shown in Tables 1 and 2.

Results

In the hydrogels of Examples 1 to 13, the adhesive force was good, the adhesive variation was also suppressed, and the handling and the processability were also excellent. In addition, the stability of the polymerization solutions was also good.
In Comparative Example 1, the dispersibility (liquid stability) of the polymerization solution was poor, and phase separation occurred, so that a hydrogel could not be prepared. The hydrogels of Comparative Examples 2 and 10 were poor in adhesive force. The hydrogels of Comparative Examples 3 to 5, 7, and 9 had large adhesive variation. The hydrogel of Comparative Example 6 did not contain fiber or an intermediate substrate, and thus had poor handling. Since Comparative Example 8 had poor dispersibility and was phase-separated, no hydrogel was produced.

[Table 1]

| | | | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|
| Composition ratio (% by mass) | Monofunctional monomer | Acrylamide | 20.0 | 20.0 | 20.0 | 20.0 | 20.0 | 20.0 | 20.0 | 19.0 | - | 20.0 | 20.0 | 20.0 | 20.0 |
| | | Acrylic acid | - | - | - | - | - | - | - | - | 14.4 | | | | |
| | | TBAS | - | - | - | - | - | - | - | - | 9.6 | | | | |
| | Crosslinkable monomer | Methylenebisacrylamide | 0.018 | 0.018 | 0.018 | 0.018 | 0.018 | 0.018 | 0.018 | 0.018 | 0.018 | 0.018 | 0.018 | 0.018 | 0.018 |
| | Water | Ion exchange water | 18.0 | 18.0 | 18.0 | 18.0 | 18.0 | 18.0 | 18.0 | 28.0 | 17.3 | 24.0 | 18.0 | 18.0 | 18.0 |
| | Humectant | Glycerol | 58.0 | 58.3 | 57.5 | 57.0 | 58.3 | 57.5 | 57.0 | 44.9 | 46.7 | 51.5 | 58.0 | 57.0 | 57.5 |
| | Electrolyte | Sodium chloride | 2.0 | 2.0 | 2.0 | 2.0 | 2.0 | 2.0 | 2.0 | 5.6 | 2.0 | 2.0 | 2.0 | 2.0 | 2.0 |
| | Base | 50% NaOH | - | - | - | - | - | - | - | - | 8.0 | - | - | - | - |
| | Fiber | Dietary fiber | Microfibrillated cellulose | 0.50 | 0.20 | 1.0 | 1.5 | 0.50 | 0.50 | 0.50 | 0.50 | 0.50 | 1.5 | - | - | - |
| | | | | - | - | - | - | - | - | - | - | - | - | 0.5 | 1.5 | 1.5 |
| | | Polyethylene fiber | 0.50 | 0.50 | 0.50 | 0.50 | 0.20 | 1.0 | 1.5 | 1.0 | 0.50 | - | 0.5 | 0.5 | - |
| | Other additives | | 0.982 | 0.982 | 0.982 | 0.982 | 0.982 | 0.982 | 0.982 | 0.982 | 0.982 | 0.982 | 0.982 | 0.982 | 0.982 |
| Total | | | 100.0 | 100.0 | 100.0 | 100.0 | 100.0 | 100.0 | 100.0 | 100.0 | 100.0 | 100.0 | 100.0 | 100.0 | 100.0 |
| Presence or absence of polyester nonwoven fabric | | | Absence | Absence | Absence | Absence | Absence | Absence | Absence | Absence | Absence | Absence | Absence | Absence | Absence |
| Thickness (mm) | | | 0.75 | 0.75 | 0.75 | 0.75 | 0.75 | 0.75 | 0.75 | 0.60 | 0.75 | 0.75 | 0.75 | 0.75 | 0.75 |
| Evaluation results | Bakelite adhesive force (N) | | 7.4 | 8.2 | 4.8 | 2.8 | 8.1 | 4.1 | 3.5 | 5.3 | 5.8 | 2.3 | 6.9 | 3.5 | 3.1 |
| | Coefficient of variation CV value (%) | | 2.1 | 2.8 | 1.9 | 1.5 | 2.9 | 3.1 | 2.6 | 2.3 | 2.0 | 1.4 | 2.4 | 2.0 | 1.6 |
| | Handling | | A | A | A | A | A | A | A | A | A | B | A | A | B |
| | Processability | | A | A | A | A | A | A | A | A | A | A | A | A | A |
| | Storage modulus (Pa) | | 5256 | 4550 | 6330 | 8100 | 4810 | 6460 | 7892 | 5640 | 4990 | 6010 | 4980 | 7020 | 6320 |
| | Liquid stability | | A | A | A | A | A | A | A | A | A | A | A | A | A |

EP 4 501 364 A1

14

[Table 2]

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Composition ratio (% by mass) | Monofunctional monomer | Acrylamide | 20.0 | 20.0 | 20.0 | 20.0 | 20.0 | 20.0 | - | 20.0 | 20.0 | 20.0 |
| | | Acrylic acid | - | - | - | - | - | - | 14.4 | - | - | - |
| | | TBAS | - | - | - | - | - | - | 9.6 | - | - | - |
| | Crosslinkable monomer | Methylenebisacrylamide | 0.018 | 0.018 | 0.018 | 0.018 | 0.018 | 0.018 | 0.018 | 0.018 | 0.018 | 0.018 |
| | Water | Ion exchange water | 18.0 | 24.0 | 18.0 | 18.0 | 18.0 | 18.0 | 17.3 | 18.0 | 18.0 | 18.0 |
| | Humectant | Glycerol | 58.6 | 50.2 | 58.6 | 58.7 | 59.0 | 59.0 | 47.7 | 58.6 | 58.7 | 56.0 |
| | Electrolyte | Sodium chloride | 2.0 | 2.0 | 2.0 | 2.0 | 2.0 | 2.0 | 2.0 | 2.0 | 2.0 | 2.0 |
| | Base | 50% NaOH | - | - | - | - | - | - | 8.0 | - | - | - |
| | Fiber | Dietary fiber | Microfibrillated cellulose | 0.40 | 2.8 | - | 0.10 | - | - | - | - | - | - |
| | | | | - | - | - | - | - | - | - | 0.40 | 0.10 | 3.00 |
| | | Polyethylene fiber | - | - | 0.40 | 0.20 | - | - | - | - | 0.20 | - |
| | Other additives | | 0.982 | 0.982 | 0.982 | 0.982 | 0.982 | 0.982 | 0.982 | 0.982 | 0.982 | 0.982 |
| Total | | | 100.0 | 100.0 | 100.0 | 100.0 | 100.0 | 100.0 | 100.0 | 100.0 | 100.0 | 100.0 |
| Presence or absence of polyester nonwoven fabric | | | Absence | Absence | Absence | Absence | Presence | Absence | Presence | Absence | Absence | Absence |
| Thickness (mm) | | | 0.75 | 0.75 | 0.75 | 0.75 | 0.75 | 0.75 | 0.75 | 0.75 | 0.75 | 0.75 |
| Evaluation results | Bakelite adhesive force (N) | | - | 0.6 | 6.1 | 7.8 | 8.9 | 9.4 | 7.0 | - | 7.3 | 0.9 |
| | Coefficient of variation CV value (%) | | - | 1.2 | 5.8 | 5.1 | 6.9 | 1.6 | 7.2 | - | 4.8 | 2.1 |
| | Handling | | - | A | A | B | A | C | A | - | B | A |
| | Processability | | - | A | A | B | A | B | A | - | B | A |
| | Storage modulus (Pa) | | - | 9900 | 4390 | 3900 | 4050 | 2095 | 3830 | - | 3550 | 8760 |
| | Liquid stability | | C | A | A | B | A | A | A | C | B | A |

**EP 4 501 364 A1**

**Claims**

1. A hydrogel comprising a hydrogel and fibers dispersed in the hydrogel,

   the hydrogel comprising no intermediate substrate, and
   the hydrogel having an adhesive force to a Bakelite plate of 1.0 to 10 N/20 mm and a coefficient of variation CV value of the adhesive force of less than 4.0.

2. The hydrogel according to claim 1, wherein the fiber contains dietary fiber.

3. The hydrogel according to claim 1, wherein the fiber contains dietary fiber and polyolefin fiber.

4. The hydrogel according to claim 2, wherein a content of the dietary fiber is 0.20 to 2.0% by mass in terms of pure content.

5. The hydrogel according to claim 2, wherein the dietary fiber has an average fiber diameter of 10 to 500 nm.

6. The hydrogel according to claim 2, wherein the dietary fiber contains cellulose.

7. The hydrogel according to claim 3, wherein a content of the polyolefin fiber is 0.20 to 2.0% by mass in terms of pure content.

8. The hydrogel according to claim 3, wherein the polyolefin fiber has an average fiber length of 0.50 to 2.0 mm.

9. The hydrogel according to claim 1, which has an adhesive force to a Bakelite plate of 2.5 to 10 N/20 mm.

10. The hydrogel according to claim 1, comprising a polymer matrix, water, and a humectant, and
    the polymer matrix being a copolymer of at least one monofunctional monomer selected from the group consisting of a (meth)acrylamide monomer and a (meth)acrylic acid ester, and a crosslinkable monomer.

11. The hydrogel according to claim 1, which has a thickness of 0.20 to 2.0 mm.

12. The hydrogel according to claim 1, which has a storage modulus of 3,000 to 9,000 Pa.

13. A biological electrode hydrogel to be placed between an electrode comprising a conductive material and a skin surface, the biological electrode hydrogel comprising the hydrogel according to claim 1.

14. A method for producing the hydrogel according to claim 1, comprising irradiating a polymerizable solution with ultraviolet light having a peak intensity of 50 to 150 mW/cm$^2$, the polymerizable solution comprising a monofunctional monomer, a crosslinkable monomer, a fiber, water, a polymerization initiator, and a humectant and having a crosslinkable monomer content of 0.010 to 0.050% by mass.

<table>
<tr><td colspan="2" align="center"><b>INTERNATIONAL SEARCH REPORT</b></td><td>International application No.<br><br><b>PCT/JP2023/005602</b></td></tr>
</table>

**A. CLASSIFICATION OF SUBJECT MATTER**

*A61K 50/00*(2006.01)i; *C09K 3/00*(2006.01)i
FI: A61K50/00 200; C09K3/00 103L

According to International Patent Classification (IPC) or to both national classification and IPC

**B. FIELDS SEARCHED**

Minimum documentation searched (classification system followed by classification symbols)

A61K50/00; C09K3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2018-529490 A (THE JOHNS HOPKINS UNIVERSITY) 11 October 2018 (2018-10-11)<br>paragraphs [0123], [0139], [0181], example 8, fig. 1, 10 | 1-14 |
| Y | JP 2021-104586 A (SEKISUI PLASTICS CO., LTD.) 26 July 2021 (2021-07-26)<br>paragraph [0062] | 1-14 |
| Y | LOPERGOLO, L. C. et al. Development of a Poly(N-vinyl-2-pyrrolidone)/Poly(ethylene glycol) Hydrogel Membrane Reinforced with Methyl Methacrylate-Grafted Polypropylene Fibers for Possible Use as Wound Dressing. Journal of Applied Polymer Science, 2020, vol. 86, pp. 662-666<br>abstract | 1-14 |
| Y | HUANG, Shu et al. Nanocellulose reinforced P(AAm-co-AAc) hydrogels with improved mechanical properties and biocompatibility. Composites Part A, 2018, pp. 395-404, https://doi.org/10.1016/j.compositesa.2018.06.028<br>abstract | 1-14 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **20 April 2023** | **09 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/005602**

| Patent document cited in search report | | | Publication date (day/month/year) | Patent family member(s) | | | Publication date (day/month/year) |
|---|---|---|---|---|---|---|---|
| JP | 2018-529490 | A | 11 October 2018 | US 2018/0243480 A1 paragraphs [0183], [0199], [0241], example 8, fig. 1, 10 WO 2017/031169 A1 EP 3337426 A1 | | | |
| JP | 2021-104586 | A | 26 July 2021 | US 2021/0198403 A1 paragraph [0071] | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020097216 A
- JP 2803886 B